# EUROPEAN PATENT SPECIFICATION

(11) **EP 0 343 223 B1**
(45) Date of publication and mention of the grant of the patent: **30.06.1993**
(21) Application number: 89900211.7
(22) Date of filing: 21.11.1988
(51) Int. Cl.: A61K 31/40, C07D 209/14

(54) **USE OF MELATONIN OR DERIVATIVES THEREOF FOR THE PRODUCTION OF PHARMACEUTICAL COMPOSITIONS EFFECTIVE TO COUNTERACT THE EFFECTS OF AGING**
VERWENDUNG VON MELATONIN ODER SEINE DERIVATE ZUR HERSTELLUNG VON ARZNEIMITTELN, DIE GEGEN ALTERSKRANKHEITEN WIRKSAM SIND
EMPLOI DE LA MELATONINE OU DE SES DERIVES POUR LA PRODUCTION DE COMPOSITIONS PHARMACEUTIQUES EFFICACES POUR CONTRECARRER LES EFFETS DU VIEILLISSEMENT

(30) Priority: 19.11.1987 GB 8727099
(43) Date of publication of application: 29.11.1989
(73) Proprietor: CELLENA (CELL ENGENEERING) A.G., CH-8123 Ebmatingen (CH)
(72) Inventor: PIERPAOLI, Walter, CH-6572 Quartino-Magadino (CH); MAESTRONI, Giorgio, I-28052 Cannobio (IT)
(74) Representative: Gutmann, Ernest
(86) International application number: EP8801059
(87) International publication number: WO8904659

(56) References cited:
- WO-A-86/05093
- GB-A- 1 493 941
- US-A- 3 642 994
- Neuroimmunomodulation, 1st Stromboli Conf. Annals of New York Acc. Sci., vol. 521, 1988, pages 140-148.
- Immunology Letters, vol. 16, December 1987 1987 W. Pierpaoli et al.: "Melatonin: a principal neuroimmunoregulatory and anti-stress hormone: its anti-aging effects", see pages 355-362; see particularly pages 358, 360
- Maestroni et al. 15th Inter. Congress of Chemoth. Istambul 1987

## Description

The present invention relates to a composition for systemic treatment to delay aging and prevent, reverse or delay the symptoms of age related debility, disease and cosmetic decline. More particularly the invention provides a composition for clinically treating the physiologic changes of senescence and degenerative disease in man and other mammals by the administration of a compositon containing a melatonin compound or homologue thereof in order to produce clinical improvement or maintain functional status in man and animals beset by age related loss in function.

More particularly, this invention provides a composition for the physiological, biochemical, immunological and cosmetic improvement of biomarkers which show evidence of decline with age. The use of melatonin, through stimulation of immunologic response and repair mechanisms should serve to increase resistance to infection and to degenerative diseases that are seen with progressive advanced chronologic age.

While aging is a natural event that leads to loss of vigour, cosmetic change, debility and death, the rate of physiologic change that leads to the expression of age related events can be slowed by environmental and nutritional manipulation. In invertebrates and cold blooded vertebrates (poikilotherms), decreasing temperature or activity which slows metabolic events delays aging. In mammals, dietary restriction in rats results in prolongation of both median and absolute survival and can restore protein synthetic and immunologic function to a more youthful range. Similar results with rejuvenation of functional decline have been obtained in older rats and mice subjected to hypophysectomy with hormonal maintenance provided by thyroid and corticosteroids.

Age related Physiological processes and biochemical processes have been reviewed in a recent paper by R. ROZENCWAIG, B.R. GRAD, and J. OCHOA in "MEDICAL HYPOTHESES" (1987), 23, 337-352 edited by the Longman Group UK Ltd.

They recall that of this time nearly fifty enzyme induction impairments have been shown to occur during senescence (ADELMAN, R.C., "Age-dependent effects in enzyme induction - A biochemical expression of aging". Exp. Geront. 6:75, 1971). These processes are responsive to various pharmacological and nutritional changes (ADELMAN R.C., FREEMAN, C. and COHEN, B.S., "Enzyme adaptation as a biochemical probe of development and aging". In: Advances in Enzyme Regulation, Weber, G. Ed. Volume 10, Oxford and New York, Pergamon Press, 365, 1972). Critical among these for the aging process is the linear decline during senescence of the pineal enzyme, N-acetyl transferase (NAT), resulting in the decline of melatonin (REITER, R.J., RICHARDSON, B.A., JOHNSON, L.Y., FERGUSON, B.N., DINH, D.T., "Pineal melatonin rhythm: Reduction in aging Syrian hamsters". Science 210:1372, 1980).

Ideas were presented by the authors, as well as others earlier, that the pathophysiological changes associated with the aging process were connected with a decline of pineal activity, particularly pineal melatonin. Thus aging of the pineal gland itself would lead to a predictable chain of events responsible for the phenomena of aging. Having recalled that "pineal rhythmicity is the only biological clock synchronized with a time dimension, which also has the capacity to repair and rejuvenate the organism" and "since the pineal gland's action to delay development is known, it is not surprising that it would also act to delay developmental senescent changes and extend the lifespan". They concluded that a possibility of the reversal of senescence called for exploration and that "this may require replacement of aelatonin along with other hormones in order to achieve a more youthful endocrine balance and homeostasis, and consequently a possible repair of the body as a whole".

USP 3,642,994 mentions the use of melatonin in the control of the symtoms of epilepsy, Parkinsonism, and the psychotherapeutic management of certain mental diseases.

The International Patent Application WO 86/05093 discloses primarlily a method and composition for topically treating the skin and/or scalp of a human host. Lotions, creams or ointments containing melatonin were also presented as useful for the up-keeping of skin in good condition or for skin regeneration, particularly in an aging population.

The invention is concerned with compounds prepared for use in a treatment that will delay or reverse the aging process. This can be understood if age is seen as a distinct syndrome governed by a neuroendocrine clock with beyond puberty and sexual maturity programs the onset of debility, impaired functional capacity, change in appearance, and degenerative disease leading to dependency and death.

This invention utilizes melatonin or related compounds to reset the neuroendocrine clock that governs aging. Melatonin even when given alone in appropriate circadian fashion, parenterally, or preferably orally or rectally, stimulates immune and cellular responses involved in homeostatic and eustatic repair mechanisms that prolonge the physiologic integrity of the body. Related homologues of melatonin, as defined hereinafter, are deemed to possess the same properties.

Melatonin and related homologues thus appear as biological transducers which alter neuroendocrine, neurohypophyseal (pituitary) function, which prolongs the physiological juvenile state and maintains youthful function and vigour beyond the median or absolute limits of the usual pattern of age related decline in function and survival. The pharmacological assays whose results are reported hereafter provide evidence that restoration of melatonin levels in vivo to their circadian values by external administration thereof under the above indicated conditions, particularly at a time between sunset and that where the subject goes to sleep, also entails the prolongation of the period of effective sexual maturity -- thereby delaying senescence, cosmetic change, debility and death. By further stimulating immune responsiveness and resistance to disease, thus blocking the entry of acute and chronic infection and associated degenerative diseases which occur with increasing chronologic age, melatonin and related homologues appear as efficient products for delaying the onset of senescence and prolonging the period of youthful survival.

The invention thus relates to the preparation of drug compositions which contain melatonin or related homologues for use in a systemic treatment utilizing melatonin and related homologues that will delay, attenuate and remit functional impairment or cosmetic changes associated with advanced chronological age.

Particularly melatonin is suitable as an active principle of a prophylactic or therapeutical drug in all age-related diseases, in particular in those syndromes and derangements consequent to age-dependent alterations of central or peripheral neuroendocrine functions like, e.g., hypothyroidism, hypercholesterolemia, senile diabetes, arteriosclerosis, hypertension,hypogonadism, hypocorticosurrenalism, post-menopausal or senile osteoporosis, anemias, immunological deficiency in responding to bacterial and viral pathogens. These syndromes represent the so-called "metabolic aging" which can be delayed by administration of exogenous melatonin to enhance the levels of melatonin at night in the treated subjects.

The invention thus relates to the production of pharmaceutical compositions for use as anti-aging agents, whose active component consists of melatonin itself. The invention also contemplates the use of chemical homologues for the same purpose. There should be mentioned more particularly the class of compounds which can be represented by the general formula : 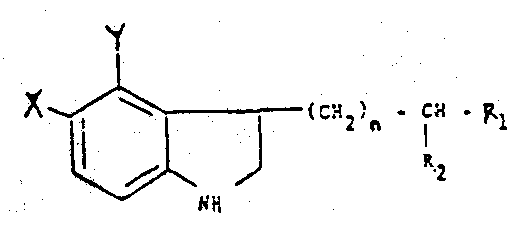
in which :
- n is 1 or 2,
- R₁ and R₂, identical or different from each other, are -H, -NH₂, -COOH, -OH, acyl, -NH-acyl or alkoxy, said acyl or said alkoxy comprising from 1 to 4 carbon atoms,
- X is -OH or alkoxy comprising from 1 to 4 carbon atoms,
- Y is -H, -OH or -NH₂.

Preferred compounds for use in the compositions of the invention are those in which Y is hydrogen and X is methoxy. The most preferred compound is melatonin itself, the formula of which is

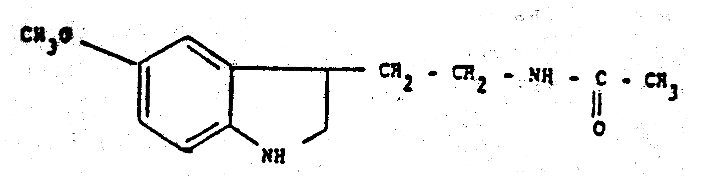

Other preferred compounds for use in the compositions of the invention are :
- 5-methoxytryptamine,
- 5-methoxytryptophan,
- 5-methoxytryptophol,
- 5-methoxyindole-3-acetic acid,
- 6-hydroxy-melatonin.

These compounds can be obtained by synthetic processes, general methods of manufacture which can be derived from those published by J. SUPNIEWSKI et al, "Synthesis of melatonin 5-methoxy-N-acetyltryptamine", published in Bull. Acad. Polon. Sci. Biol., 8, pp.479-481, 1960, or Mashkovsky et al, in Farmakol. Toksikol., 26, n^{o} 1, 10, 1963, said methods being of course in each case adapted to the particular compound sought.

Further details will appear from the following disclosure of examples which establish the capability of melatonin and related homologues to effectively enhance the resistance of a host against the effects of aging, as manifested by the prolongation of life in aging mice under the conditions disclosed hereafter.

Experiments were initiated in 1985 in which groups of C57BL/6J inbred male mice, aged 575 days (10 mice per group), were given melatonin (10 µg/ml) in the drinking water while the controls received tap water containing the same amount of absolute ethanol used to prepare melatonin stock solution (1 ml ethanol in 10 liters of tap water). The drinking water with or without melatonin was given at 6 p.m. and removed at 8.30 a.m. The drinking water was changed twice weekly. Starting at 5 months after initiation of the nocturnal, oral melatonin treatment, when the mice reached two years of age, a striking difference of body weight and of general body and pelage conditions started to be evident. Although some of the mice died earlier in both groups, the majority of the surviving mice treated with melatonin maintained their optimal body weight until 28 to 33 months of age (35.8± 4 grams) while the untreated mice lost progressively weight and were all dead within 29 months of age. Average body weight of the control mice at 27 months of age was 27.8± 4 grams.

The last melatonin-treated mouse died at 3 years of age. These date though preliminary make it evident that melatonin treatment of aging mice prolonged their life or circa 20 percent (average 6 months longer). The life span of melatonin-treated mice was 931 ± 80 days while that of control mice was 755 ± 81 days (p < 0.01).

These remarkable effects of cyclical, chronic oral administration of melatonin alone on the aging process and their dimension surpassed any expectation one could have had upon initiating these assays. In fact, evidently and as judged by the prolonged objective, youthful appearance of the mice (vigour, fur quality and brightness, motility, weight), melatonin not only prolonged the life of the animals, but also exerted an extraordinary positive action on their performance and reversed or delayed the symptoms of age-related debility, disease and cosmetic decline in a dramatic fashion.

It is therefore striking that melatonin alone administered exogeneously appears to act as a substantial substitute for the highly complex functions of the pineal gland which are reduced in a living host under the effects of aging, and in particular that exogenous melatonin appears capable of slowing down the progressive decline of absolute or cyclic melatonin synthesis in the pineal gland consequent to a variety of factors involving its metabolic precursors and/or enzymatic pathway leading, from serotonin, to its optimal and individually-shaped night release. This "metabolic aging" may in fact be corrected and even reversed by exogenous chronic, cyclic restoration of an optimal night periodicity of melatonin level in the aged, as shown in rejuvenated mice of the above disclosed experiment.

The additional data hereafter demonstrate the regulatory effects of systemic, exogenous melatonin on the regulation of the hypothalamic-pituitaru-adrenal-gonadal-thyroid axes.

It can be seen (Table 1) that chronical treatment with melatonin in the drinking water in aging mice elevates the levels of T3 (tetraiodotyronine) and T4 (tyroxine) in their peripheral blood and corrects thus ageing-related thyroid dysfunction or lowered function.

Table 2 shows that levels of cholesterol, triglycerides and phospholipids are significantly increased in the peripheral blood of 23 month-old, aging C57BL/6 mice which had been pinealectomized at the age of four months. Also the chronical treatment with night melatonin in aging mice corrects the aging-related increase of lipids in blood (not shown here). It follows that the increase of the production of thyroid hormones induced in vivo by exogenous melatonin should also be accompanied by an improved degradation of cholesterol, triglycerides and phospholipids and by detoxification effects at the liver level, all these beneficial effects resulting in a slowing down of the aging process.

**TABLE 1**

| Chronical night treatment with melatonine in the drinking water elevates levels of T3 and T4 and of T4/T3 ratio in the blood of aging mice. | | | | | | |
|---|---|---|---|---|---|---|
| Strain of mice | No | Age * (months) | Months of melatonin treatment | T3 | T4 | T4/T3 |
| C57BL/6 female untreated | 9 | 22 | - | 0.614± 0.186 | 30.00± 14.03 | 48.86 |
| C57BL/6 female melatonin-treated | 9 | 22 | 5 | 0.691± 0.077 (+11%) | 37.50 5.65 (+20%) | 54.27 (+10%) |
| C3H/He female untreated | 7 | 23 | - | 0.531± 0.225 | 18.5± 8.1 | 34.84 |
| C3H/He female melatonin-treated | 6 | 23 | 3 | 0.664± 0.137 (+25%) | 28.2± 0.8 (+34%) | 42.47 (+18%) |
| Details are indicated in the text. The mice were bled at 11 am from theretroorbital plexus under acute ether anaesthesia. T3 and T4 was measured by radioimmunoassay in the serum of individual mice. | | | | | | |

| | | | | | | |
|---|---|---|---|---|---|---|
| * When bleeding and measurements of T3 and T4 were performed. | | | | | | |

**TABLE 2**

| Pinealectomy produces an increase of lipid levels in the peripheral blood of aging C57BL/6 female mice. | | | | | | |
|---|---|---|---|---|---|---|
| Groups | No of mice | Age* (months) | Months after pinealectomy | Cholesterole (nmol/1) | Triglycerides (nmol/1) | Phospholipids (nmol/1) |
| Pinealect. | 8 | 23 | 19 | 1.91 ± 0.35 (+30%) | 1.03 ± 0.28 (+26%) | 2.21 ± 0.27 (+21% |
| Shamopereated | 6 | 23 | 19 | 1.47 ± 0.11 | 0.82 ± 0.13 | 1.82 ± 0.60 |

| | | | | | | |
|---|---|---|---|---|---|---|
| * When bleeding and measurements of lipids were performed | | | | | | |

Thus melatonin and related homologues may particularly be used to combat or slow down the effects of aging, accordingly aging itself.

The invention thus concerns pharmaceutical compositions containing melatonin, or an homologue thereof, prepared with a view of preventing aging in mammals, such pharmaceutical compositions further containing a pharmaceutical vehicle suitable for the chosen administration route. These compositions may of course also include other active principles which are likely, in association or synergetically, to interact with melatonin or the relevant homologue to achieve the effect sought.

Effective use of melatonin or its chemical homologues with a view of reinforcing the defences of the mammal host against aging, comprises administering to the latter an effective dose of said melatonin or homologues, in one of the administration forms which have been mentioned above, preferably from sunset, or a little earlier, say from 4 p.m. up to the time where the host goes to sleep. Examples of daily doses capable of inducing an effect, range from 0.1 to 200 mg, preferably from 0.5 to 50 mg per kg of body weight, when the administration is effected by the oral or rectal route, or again from 0.05 to 10 mg, preferably from 1 to 5 mg per kg of body weight, when administered by the parenteral route.

These unit doages are of course only indicative as the actual dosages should be adjusted by the clinician for each person individually, among other factors according to his own age and degree of clinical senescence. Administrations may be chronical or cyclic over periods, e.g. from one to four weeks separated by time intervals without treatment.

## Claims

1. Use of a compound hereafter defined for the production of pharmaceutical compositions for use in the systemic treatment or prevention of the effects of metabolic aging, wherein said compound has the following formula : 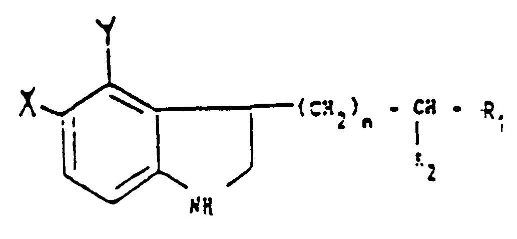 in which :
- n is 1 or 2,
- R₁ and R₂, identical or different from each other, are -H, -NH₂, -COOH, -OH, acyl, -NH-acyl or alkoxy, said acyl or said alkoxy comprising from 1 to 4 carbon atoms,
- X is -OH or alkoxy comprising from 1 to 4 carbon atoms,
- Y is -H, -OH or -NH₂.

2. The use of claim 1, wherein said compound is melatonin.

3. The use of claim 1 or 2 which is for use for the production of pharmaceutical compositions suitable for the systemic treatment or prevention of the effects of metabolic aging by inducing in vivo an increase of thyroid hormones and an improved degradation and detoxification of cholesterol, triglycerides and phospholipids at the level of liver.

## Patentansprüche

1. Verwendung einer der nachstehend definierten Verbindungen zur Herstellung von Arzneimitteln zur Verwendung in der Behandlung von oder zum Vorbeugen vor Wirkungen metabolischen Alterns in Organismen, wobei die Verbindung die folgende Formel aufweist: 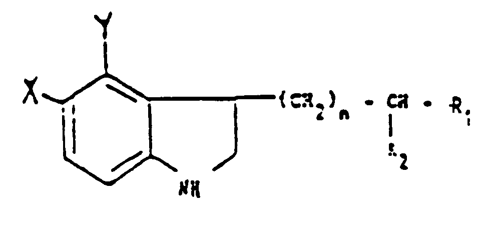 in der
n der Wert 1 oder 2 ist,
R₁ und R₂, entweder identisch oder voneinander verschieden, die Reste -H, -NH₂, -COOH, -OH, Acyl-, -NH-Acyl- oder Alkoxy- darstellen, und die Acyl- oder Alkoxyreste 1 bis 4 Kohlenstoffatome umfassen,
X der Rest -OH oder ein Alkoxyrest umfassend 1 bis 4 Kohlenstoffatome ist,
Y der Rest -H, -OH oder -NH₂ ist.

2. Verwendung nach Anspruch 1, wobei die Verbindung Melatonin ist.

3. Verwendung nach Anspruch 1 oder 2, nämlich zur Verwendung in der Herstellung von Arzneimittel, geeignet in der Behandlung von oder zum Vorbeugen vor Wirkungen metabolischen Alterns in Organismen durch die Induktion eines Anstiegs an Thyroid-Hormonen in vivo und einem verbesserten Abbau und Entgiftung von Cholesterin, Triglyceriden und Phospholipiden in bezug auf die Leberwerte.

## Revendications

1. Utilisation d'un composé comme défini ci-après pour la production de compositions pharmaceutiques pour l'utilisation dans le traitement ou la prévention des effets du vieillissement métabolique, dans laquelle ledit composé a la formule : 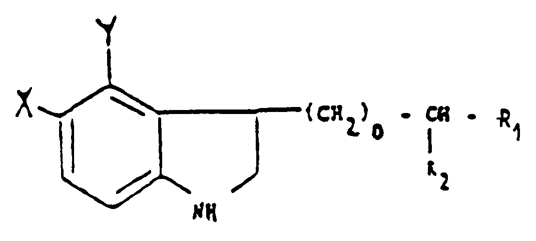 dans laquelle :
- n est 1 ou 2 ;
- R₁ et R₂, identiques ou différents l'un de l'autre, sont -H, -NH₂, -COOH, -OH, acyle, -NH-acyle ou alcoxy, cet acyle ou cet alcoxy comprenant de 1 à 4 atomes de carbone ;
- X est -OH ou alcoxy comprenant de 1 à 4 atomes de carbone ;
- Y est -H, -OH ou -NH₂.

2. L'utilisation selon la revendication 1, dans laquelle ce composé est la mélatonine.

3. L'utilisation selon la revendication 1 ou 2 qui est pour la production de compositions pharmaceutiques adaptées au traitement ou à la prévention systémiques des effets du vieillissement métabolique par induction in vivo d'un accroissement des hormones thyroïdiques et d'une dégradation et détoxication améliorées du cholestérol, des triglycérides et phospholipides au niveau du foie.
